# EUROPÄISCHE PATENTANMELDUNG

(11) **EP 1 136 090 A2**
(43) Veröffentlichungstag der Anmeldung: **26.09.2001**
(21) Anmeldenummer: 01105729.6
(22) Anmeldetag: 08.03.2001
(51) Int. Cl.: A61M 5/172

(54) **Narkoseregler**

(30) Priorität: 17.03.2000 DE 20005004 U
(71) Anmelder: B. BRAUN MELSUNGEN AG, 34212 Melsungen (DE)
(72) Erfinder: Heitmeier, Rolf, 34225 Baunatal (DE); Niessner, Gerhard, 34212 Melsungen (DE)
(74) Vertreter: Selting, Günther, Dipl.-Ing.

(57) **Zusammenfassung**

Zur automatischen Zufuhr eines Wirkstoffes zu einem Patienten (14) wird die Förderrate (R) einer Infusionspumpe (13) ausgewertet. Die Förderrate wird in einem Patientenmodell (11) verarbeitet, welches die entsprechenden Wirkstoffdaten von einer Medikamentendatei (12) empfängt. Das Patientenmodell (11) errechnet aus den zurückliegenden Werten der Wirkstoffzufuhr die Konzentration (CNᵢₛₜ) im Patientenkörper. Diese Ist-Konzentration wird einem Konzentrationsregler (17) zugeführt, der ferner eine Soll-Konzentration (CNₛₒₗₗ) empfängt. In Abhängigkeit von der Differenz wird die Infusionspumpe (13) gesteuert. Die Soll-Konzentration an einer Eingabevorrichtung (19) manuell eingestellt werden, oder sie wird von einem weiteren Regelteil geliefert, welches aus einem EEG des Patienten z.B. ein BIS-Niveau erzeugt, welches eine Angabe über die Narkosetiefe darstellt.

## Beschreibung

Die Erfindung betrifft einen Narkoseregler zur Beeinflussung der Rate einer Wirkstoffzufuhr zu einem Patienten zur Erzielung und Aufrechterhaltung eines gewünschten Narkosezustandes.

Geschlossene Regelkreise für Anästhesien werden seit längerer Zeit erforscht. Die Pharmaforschung hat grundlegende Wirkmodelle von spezifischen Wirkstoffen entwickelt. Diese Erkenntnisse erlauben eine mathematische Nachbildung der Wirkstoffanreicherung im Blutplasma des Patienten. Die Vorhersagegenauigkeit beträgt etwa +/-30%. Neueste Anwendungen erlauben eine modellbasierte Regelung von spezifischen Wirkstoffen. Je nach Handlingsgewohnheit des Anwenders möchte man die Qualität vorhersagender Algorithmen oder die Qualität direkt regelnder Algorithmen nutzen. Wesentlich ist dabei die grundlegende Erkenntnis, dass ein empirisch ermitteltes Modell nicht die vollständigen Zusammenhänge nachbilden kann und die errechnete Konzentration nicht unbedingt mit dem Wirkeffekt und seiner Ausprägung zusammenhängen muss. In jedem Fall ist eine auf medizinischer Erfahrung basierende Korrektur durch einen Anwender, der verschiedene nicht messbare Parameter zur Beurteilung der Wirkeffektausprägung benutzt, wünschenswert.

Der Erfindung liegt die Aufgabe zugrunde, einen Narkoseregler zu schaffen, der auf der Basis der Wirkstoffkonzentration im Patientenkörper arbeitet, obwohl diese Wirkstoffkonzentration als Messgröße nicht in Ist-Zeit erfassbar ist.

Die Lösung dieser Aufgabe erfolgt erfindungsgemäß mit den im Anspruch 1 angegebenen Merkmalen. Hiernach wird die gegenwärtige Wirkstoffkonzentration im Patientenkörper anhand eines Patientenmodells unter Berücksichtigung zurückliegender Werte der Wirkstoffzufuhr berechnet. In Abhängigkeit von dem auf diese Weise errechneten aktuellen Wert der gegenwärtigen Wirkstoffkonzentration wird in dem Narkoseregler die Rate der Wirkstoffzufuhr derart verändert, dass die gegenwärtige Wirkstoffkonzentration auf einen Zielwert eingeregelt wird. Auf diese Weise erfolgt mit einem Regelkreis eine Einregelung der Wirkstoffkonzentration im Patientenkörper auf einen gewünschten Zielwert. Das Patientenmodell wird im Rahmen eines vorhersagenden Algorithmus unter Berücksichtigung zurückliegender Wirkstoffzugaben betrieben, wobei zwischen unterschiedlichen Verteilungszeiträumen mit unterschiedlichen Zeitverhalten unterschieden werden kann. Das Patientenmodell liefert somit aus den vorhergehenden Wirkstoffraten und Zufuhrdauern eine Angabe über die gegenwärtige Wirkstoffkonzentration im Patientenkörper. Diese Berechnung ist vorhersagend und wird als TOI-Verfahren bezeichnet (Target Oriented Infusion). Der zur Errechnung der Wirkstoffkonzentration erforderliche Parametersatz wird aus einer Medikamentendatenbank entnommen und an das Patientenmodell übergeben. Das Patientenmodell errechnet die sich aus dem Profil der vergangenen Wirkstoffzufuhr ergebende Wirkstoffkonzentration, wobei dieser Wert als Ist-Wert für die Regelung benutzt werden kann.

Der Zielwert der Wirkstoffkonzentration kann vom Benutzer an einer Eingabevorrichtung einstellbar sein. Dies ermöglicht es, ärztliches Wissen in die Regelung einzubringen. Der überwachende Arzt kann anhand eines Zeitdiagramms der Wirkstoffkonzentration im Patientenkörper einen Zielwert festsetzen und diesen einstellen. Dabei kann er auch zeitliche Änderungen des Zielwertes durchführen.

Gemäß einer bevorzugten Weiterbildung der Erfindung wird der Regelteil für die Wirkstoffkonzentration ergänzt durch einen BIS-Regelteil. Dieser liefert aus dem Bispektralindex einen jeweiligen Zielwert der Wirkstoffkonzentration. Der Bispektralindex ist eine aus dem EEG des Patienten abgeleitete Kenngröße, die gewissermaßen den Narkosepegel angibt. Der Index besteht aus Werten zwischen 0 (hellwach) und 100 (sediert). Anhand des jeweiligen BIS-Wertes kann eine manuelle Verstellung des Zielwertes der Wirkstoffkonzentration erfolgen. Es ist aber auch möglich, den BIS-Wert für eine automatische Einstellung des Zielwertes der Wirkstoffkonzentration zu benutzen. Vorzugsweise wird der zeitliche Verlauf des BIS-Wertes an einem Display angezeigt. Der Anwender kann somit zu jedem Zeitpunkt ein vollständiges Bild der Wirkstoffzufuhr, der Wirkstoffkonzentration und der Narkosetiefe erhalten.

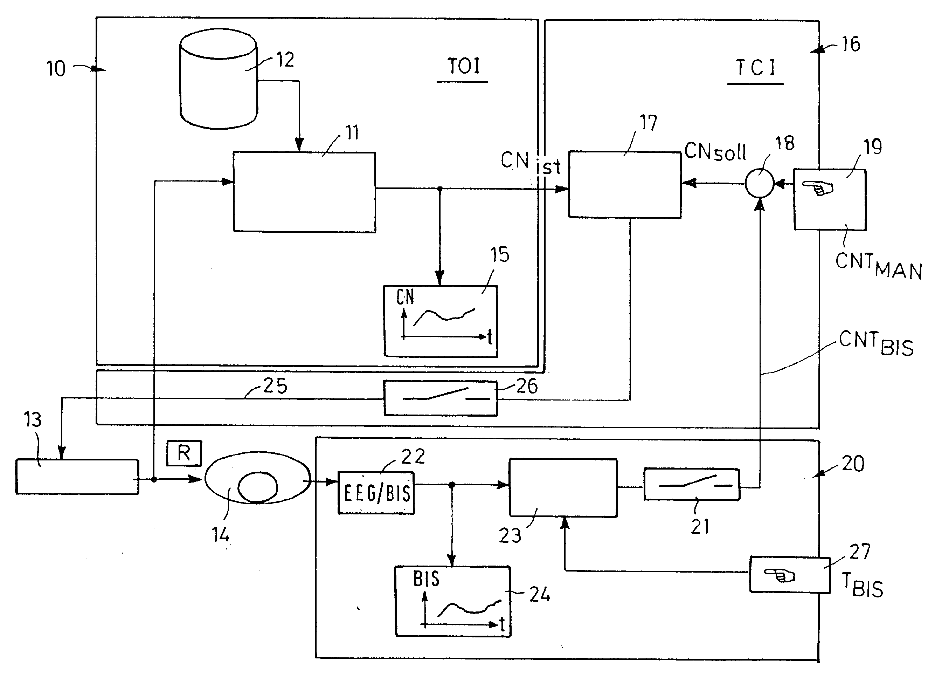

Im folgenden wird unter Bezugnahme auf die einzige Figur der Zeichnung ein Ausführungsbeispiel der Erfindung näher erläutert.

In der Zeichnung ist ein Blockschaltbild des Narkosereglers dargestellt.

Der Narkoserechner weist einen Modell-Rechenteil 10 auf, welcher ein Patientenmodell 11 enthält. Das Patientenmodell ist eine rechnerische Nachbildung der Reaktion des Patientenkörpers auf Wirkstoffe. Die Daten zahlreicher Wirkstoffe sind in einer Medikamentendatei 12 enthalten. Das Patientenmodell 11 ist ein komplexes Modell, das sich aus drei Verteilungsräumen mit unterschiedlichen Zeitverhalten zusammensetzt. Das Patientenmodell empfängt von der Medikamentendatei 12 nicht nur die Wirkstoffdaten sondern auch die in dem jeweiligen Medikament enthaltene Wirkstoffkonzentration.

Eine Infusionspumpe 13 liefert an den Patienten 14 eine den Wirkstoff enthaltende Infusionsflüssigkeit mit einer Zuführrate R. Der Wert der Zuführrate R wird dem Patientenmodell 11 laufend mitgeteilt.

Aus den zurückliegenden Werten der Wirkstoffzufuhr errechnet das Patientenmodell 11 den Ist-Wert der Wirkstoffkonzentration CNᵢₛₜ im Patientenkörper. Gleichzeitig wird dieser Ist-Wert der Wirkstoffkonzentration CNᵢₛₜ über der Zeit t an einem Display 15 angezeigt.

Die Wirkstoffkonzentration im Patientenkörper ist im vorliegenden Fall die Wirkstoffkonzentration im Blutplasma des Patienten. Sie wird nach dem vorhersagenden Algorithmus TOI (Target Oriented Infusion) aus den Werten der Zuführrate R ermittelt.

Der Wert CNᵢₛₜ wird einem Konzentrationsregler 17 zugeführt, der in einem Regelteil 16 enthalten ist. Der Konzentrationsregler 17 empfängt ferner den Soll-Wert der Wirkstoffkonzentration CNₛₒₗₗ von einer Selektionseinrichtung 18. Die Selektionseinrichtung 18 wählt entweder das von einer Eingabeeinrichtung 19 gelieferte manuell eingegebene Zielsignal CNT_{MAN} für die Wirkstoffkonzentration oder das von einem BIS-Regelteil 20 gelieferte Signal CNT_{BIS}. Dieses Signal wird über eine Schalteinrichtung 21 geliefert. Wenn die Schalteinrichtung 21 gesperrt ist, ist als Soll-Signal CNₛₒₗₗ das Signal CNT_{MAN} wirksam. Wenn die Schalteinrichtung 21 auf Durchlass geschaltet ist, ist nur das Signal CNT_{BIS} wirksam.

Der BIS-Regelteil 20 enthält einen EEG/BIS-Umsetzer 22, der aus den EEG-Signalen des Patienten 14 einen BIS-Pegel ermittelt, welcher ein Maß für die Narkosetiefe ist.

Der BIS-Regelteil 20 enthält ferner einen BIS-Regler 23, dem einerseits als Soll-Wert ein BIS-Target T_{BIS} zugeführt wird und andererseits das von dem EEG/BIS-Umsetzer 22 kommende BIS-Signal. Der BIS-Regler 23 erzeugt aus der Regelabweichung, also der Differenz der Signale T_{BIS} und BIS das Targetsignal für die Wirkstoffkonzentration CNT_{BIS}.

In dem BIS-Regelteil wird die Höhe des BIS-Pegels über der Zeit t an einem Display 24 angezeigt.

In der Steuerleitung 25 vom Konzentrationsregler 17 zu der Infusionspumpe 13 ist eine Schalteinrichtung 26 vorgesehen. Bei leitend geschalteter Schalteinrichtung 26 übernimmt der Konzentrationsregler 17 die Steuerung der Infusionspumpe 13. Der Konzentrationsregler kann nach gängigen deterministischen Regelverfahren betrieben werden, beispielsweise als PID-Regler, Zweipunktregler o.dgl.

In Abhängigkeit von der Stellung der Schalteinrichtung 21 wählt die Selektionseinrichtung 18 aus, ob als Soll-Signal CNₛₒₗₗ das Signal CNT_{BIS} oder das Signal CNT_{MAN} zugeführt werden soll. Der Benutzer hat somit die Möglichkeit, entweder an der Eingabeeinrichtung 19 eine gewünschte Wirkstoffkonzentration CN_{MAN} einzustellen oder an einer Eingabevorrichtung 27 einen gewünschten BIS-Pegel. Bei Eingabe eines BIS-Pegels wird bei leitender Schalteinrichtung 21 die Förderrate der Infusionspumpe 13 derart geregelt, dass der gewünschte BIS-Pegel T_{BIS} eingehalten wird.

Die Kombination der PCI-Regelung mit einer BIS-Regelung hat den Vorteil, dass unabhängig von dem sich aufbauenden Bispektralindex (BIS) ein schnelles Anregeln eines ersten Konzentrationsniveaus erfolgt. Der Anwender erhält zu jedem Zeitpunkt ein vollständiges Bild der Wirkstoffzufuhr, Plasmakonzentration und der Narkosetiefe.

Zur Überwachung der automatisierten Narkose wird die therapeutische Breite der Wirkstoffzufuhr überwacht. Hierzu wird festgestellt, ob die sich einstellende Wirkstoffkonzentration zwischen einem oberen und einem unteren Grenzwert bleibt. Werden die Grenzwerte erreicht, so wird der Anwender hierauf hingewiesen, um entsprechend zu reagieren. Parallel wird der Wirkstoffverbrauch monitorisiert, der ebenfalls eine für die Narkose wichtige Information darstellt.

## Patentansprüche

1. Narkoseregler zur Beeinflussung der Rate einer Wirkstoffzufuhr zu einem Patienten (14) zur Erzielung und Aufrechterhaltung eines gewünschten Narkosezustandes, mit einem Modell-Rechenteil (10), der unter Berücksichtigung zurückliegender Werte (R) der Wirkstoffzufuhr anhand eines Patientenmodells (11) einen aktuellen Wert der gegenwärtigen Wirkstoffkonzentration im Patientenkörper (CNᵢₛₜ) errechnet, und mit einem Regelteil (16), der die Rate (R) der Wirkstoffzufuhr in Abhängigkeit von dem aktuellen Wert (CNᵢₛₜ) der gegenwärtigen Wirkstoffkonzentration derart verändert, dass die gegenwärtige Wirkstoffkonzentration (CNᵢₛₜ) auf einen Zielwert (CNₛₒₗₗ) eingeregelt wird.

2. Narkoseregler nach Anspruch 1, **dadurch gekennzeichnet, dass** der Zielwert (CNₛₒₗₗ) der Wirkstoffkonzentration an einer Eingabevorrichtung (19) einstellbar ist.

3. Narkoseregler nach Anspruch 1 oder 2, **dadurch gekennzeichnet, dass** der Zielwert (CNₛₒₗₗ) der Wirkstoffkonzentration in einem Körperfunktionsrechenteil (20) in Abhängigkeit von gemessenen Körperfunktionen festgelegt wird.

4. Narkoseregler nach Anspruch 3, **dadurch gekennzeichnet, dass** ein, der Wirkung auf die Körperfunktion adäquates Signal (z.B. BIS-Signal) verwendet wird und dass ein Regler vorhanden ist, der aus diesem physiologischen Signal einen Zielwert (CNT_{BIS}) der Wirkstoffkonzentration berechnet.

5. Narkoseregler nach Anspruch 4, **dadurch gekennzeichnet, dass** der BIS-Regler (23) einen vorgebbaren Zielwert (T_{BIS}) des Bispektralindex empfängt und aus der Regelabweichung des Bispektralindex einen Zielwert (CNT_{BIS}) der Wirkstoffkonzentration errechnet.

6. Narkoseregler nach einem der Ansprüche 1 bis 5, **dadurch gekennzeichnet, dass** eine Selektionseinrichtung (18) vorgesehen ist, welche eine Auswahl zwischen zwei separat erzeugten Zielwerten (CNT_{MAN}, CNT_{BIS}) der Wirkstoffkonzentration ermöglicht.

7. Narkoseregler nach einem der Ansprüche 1 bis 6, **dadurch gekennzeichnet, dass** der zeitliche Verlauf von Wirkstoffzufuhr (R), Wirkstoffkonzentration (CN) und/oder eine Angabe über die Narkosetiefe an einer Anzeigevorrichtung (15,24) anzeigbar ist.

8. Narkoseregler nach einem der Ansprüche 1 bis 7, **dadurch gekennzeichnet, dass** ein oberer und ein unterer Grenzwert für die Wirkstoffkonzentration vorgegeben sind.
